# EUROPEAN PATENT APPLICATION

(11) **EP 2 597 096 A1**
(43) Date of publication of application: **29.05.2013**
(21) Application number: 11382360.3
(22) Date of filing: 24.11.2011
(51) Int. Cl.: C07D 473/18

(54) **Process for preparing entecavir and intermediates thereof**

(71) Applicant: Esteve Química, S.A., 08024 Barcelona (ES)
(72) Inventor: Berenguer Maimo, Ramon, 08001 Barcelona (ES); Badia Perez, Laura, 08203 Sabadell (ES); Gasanz Guillen, Yolanda, 08025 Barcelona (ES); Velasco Turbau, Javier, 08470 Sant Celoni (ES); Ariza Piquer, Javier, 08024 Barcelona (ES)
(74) Representative: ZBM Patents

(57) **Abstract**

It comprises the preparation of entecavir of formula (I), or a pharmaceutically acceptable salt thereof, or a hydrate thereof by submitting a compound of formula (III) where X is an halogen selected from Cl, Br, and I, first to a hydrolysis reaction using formic acid and then to a deprotection reaction, and isolating the entecavir either as free base or as a pharmaceutically acceptable salt. It also comprises intermediates which intervene in this preparation process.

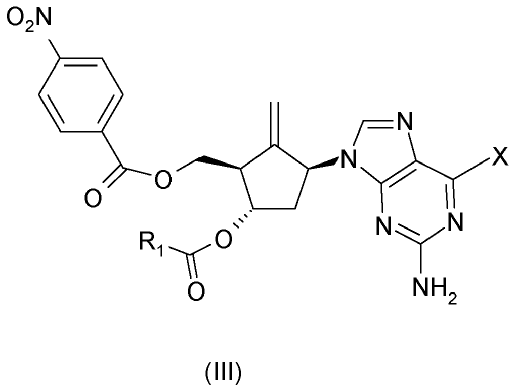

## Description

The present invention relates to a process for the preparation of an antiviral drug known as entecavir, as well as to some new intermediates useful in such preparation process.

### BACKGROUND ART

Entecavir is the International Non-proprietary Name (INN) of 2-amino-9-[(1*S*,3*R*,4*S*)-4-hydroxy-3-(hydroxymethyl)-2-methylidenecyclopentyl]-6,9-dihydro-3*H*-purin-6-one, and CAS No 142217-69-4. Entecavir is marketed as monohydrate under the trade name Baraclude by Bristol-Myers Squibb. It is administered orally in tablet form or as an oral solution containing 0.05 mg/ml. It is a nucleoside analog, more specifically, a guanine analogue, that inhibits reverse transcription, DNA replication and transcription in the viral replication process. Entecavir is used to treat chronic hepatitis B. It also helps to prevent the hepatitis B virus from multiplying and infecting new liver cells. Entecavir is also indicated for the treatment of chronic hepatitis B in adults with HIV/AIDS infection.

The structure of entecavir corresponds to formula (I).

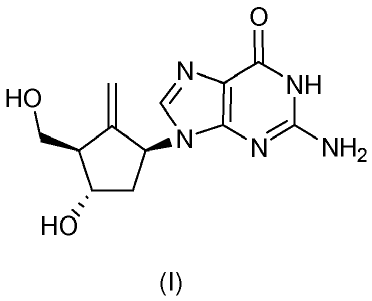

Different synthetic strategies for the preparation of entecavir and its salts are known. The reported processes are mainly focused on how to construct the chiral carbocyclic core and how to introduce the guanidine moiety to the core.

For instance, Example 1 of EP481754-A describes a process for the preparation of entecavir comprising the coupling of a protected epoxide of formula below with an O-benzyl guanine and subsequent conversion to entecavir. Thus, after introducing the guanine moiety, it is needed an oxidation and then a methylenation reaction to complete the construction of the carbocycle moiety and a deprotection reaction to yield entecavir.

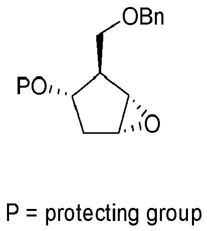

CN1566118-A describes a similar process as the one described above from a protected epoxide where the primary and secondary alcohols are forming an acetal.

Li Wei Guo et al., describe a process for the preparation of entecavir based on the use of Corey lactone diol which is transformed to the compound (1) below. This compound is reacted with O-benzyl guanine and subsequently transformed into entecavir (cf. Li Wei Guo et al., "A new route for synthesis of Entecavir", Chinese Chemical Letters 2006 vol. 17, pp. 907-910).

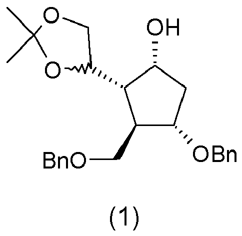

CN1861602-A describes a process for the preparation of entecavir also based on the use of the compound (1), its reaction with O-benzyl guanine and its subsequently transformation into entecavir

Other known processes are focused on the use of an intermediate olefin cyclopentane of formula (2) below, where R₁, R₂ are protective groups and R₃ is H or a protective group.

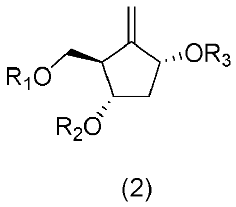

The preparation of entecavir from this intermediate cyclopentyl olefin of formula (2) comprises submitting it to a Mitsunobu reaction with a guanine precursor to yield a carbocyclic nucleoside intermediate, followed by removal of the protective groups and hydrolysis to yield entecavir.

Thus, the W0200452310-A describes several processes for the preparation of entecavir from different intermediate compounds. Some of them comprise the use of an intermediate olefin compound as the one of formula (2) where R₃ is H, and R₁ and R₂ are equal protective groups selected from silyl and carbonyl groups. This intermediate is reacted with a guanine precursor such as 2-amino-6-halopurine and the resulting carbocyclic nucleoside is converted into entecavir by treatment with a base.

WO201074534-A describes a process for the preparation of entecavir based on preparing a compound of formula (2) where R₁ and R₂ are independently H or an OH protective group, or both are forming part of a cyclic OH protective group, and R₃ is H, alkylsilyl, or allylsilyl, followed by its conversion into entecavir. According to the Example 3 of page 17 it is prepared a compound (2) where R₁ is CPh₃, R₂ is tert-butyldiphenylsilyl (TBDPS) and R₃ = H. The compound obtained is coupled with a substituted chloropurine to yield a 2-amino-6-chloropurine derivative having the hydroxyl groups protected. Then, a deprotection reaction is carried out, followed by a hydrolysis reaction using aqueous sodium hydroxide to convert the chlorine group of the deprotected 2-amino-6-chloropurine derivative into the oxo moiety of the entecavir.

CN101130542-A also discloses the conversion of the halo group of a 2-amino-6-halopurine derivative having both hydroxyl groups protected into the oxo moiety by treatment with aqueous sodium hydroxide or potassium hydroxide. The compound obtained is deprotected to yield entecavir.

The hydrolysis reaction of some 2-amino-6-halopurine derivative intermediates of entecavir has also been described using acidic conditions. CN101148450-A discloses a process for preparing entecavir where the conversion of the halo group of a 2-amino-6-halopurine derivative into the oxo moiety of the entecavir is carried out by treatment with aqueous hydrochloric acid. The reaction is disclosed either from a deprotected 2-amino-6-halopurine derivative or from a 2-amino-6-halopurine derivative having the two hydroxyl groups protected as benzyloxy.

Finally, WO201146303-A discloses a process for preparing entecavir which comprises submitting a 2-amino-6-halopurine derivative having the primary hydroxyl group at the 2-position of the cyclopentanol protected, simultaneously to a deprotection reaction of the protective group and to a hydrolysis of the halo group to yield the oxo moiety of entecavir using an acidic solution (cf. Example 1-9). Among the possible acids that can be used the following are mentioned: hydrochloric acid, sulfuric acid, trifluoroacetic acid and a mixture thereof in water.

Despite the teaching of all these prior art documents, the research of new preparation processes of entecavir is still an active field, since the industrial exploitation of known processes is difficult. Thus, the provision of an efficient and amenable way to scale-up the preparation process of entecavir is still highly desirable.

### SUMMARY OF THE INVENTION

Inventors have found a process for the preparation of entecavir with high yield and purity comprising the use of new intermediate compounds. It has been possible thanks to the inventors finding that the hydrolysis of an 2-amino-6-halopurine derivative having the primary hydroxyl at 2-position of the cyclopentane moiety protected as nitrobenzoyl ester and the secondary hydroxyl at 3-position of the cyclopentane moiety as a (C₁-C₆)-alkanoyl ester, in particular as an acetyl ester, benzoyl ester, or benzoyl ester substituted by a radical selected from the group consisting of (C₁-C₄)-alkyl, (C₁-C₄)-alkoxy, halogen, and nitro, goes in a selective manner over the 6-position of the halopurine when formic acid is used to carry out the conversion of the halo group of the protected 2-amino-6-halopurine derivative into the oxo moiety. Likewise, the subsequent deprotection of the ester of the secondary hydroxyl and of the nitrobenzoyl ester of the primary hydroxyl can be carried out using mild reaction conditions.

On the one hand, the protection of the primary hydroxyl at 2-position of the cyclopentane moiety as nitrobenzoyl ester combined with the protection of the secondary hydroxyl group as an ester from those mentioned above has resulted in that this combination confers a special strength from the possible partial deprotections of the hydroxyls during hydrolysis of the halo group into the oxo group. Thus, the hydrolysis of the halo group into the oxo group can be carried out selectively, i.e. without affecting said protective groups. Therefore, no impurities due to dimerization reactions or elimination reactions are formed, either. When the hydrolysis is carried out with other protected 2-amino-6-halopurine derivatives known in the prior art, for instance, with a 2-amino-6-halopurine derivative having both hydroxyl groups protected as acetyl ester, the selectivity is considerably lower obtaining important quantities of by-products of partial deprotection of the acetyl groups. Although these by-products can be transformed into entecavir, the low selectivity of the reaction does not allow isolating any compound with a good yield.

On the other hand, the selection of formic acid has resulted to be a selective reagent for carrying out the hydrolysis of the halo group into the oxo group without affecting the OH protective groups when the protective groups mentioned above are present. The use of formic acid also helps to dissolve the protected 2-amino-6-halopurine derivative in a controlled reaction conditions.

The inventors have found that when the hydrolysis of the halo group into the oxo group is carried out with diluted hydrochloric acid as in the prior art, no reaction occurred. When the reaction conditions were forced using more concentrated hydrochloric acid at higher temperatures, the hydrolysis was not selective and deprotection also took place, thus yielding important amount of impurities. Attempts to carry out the hydrolysis and deprotection steps together using sodium hydroxide as it is disclosed in the prior art, gave important amounts of dimerization and elimination by-products.

The process of the present invention considerably diminishes the formation of by-products of the hydrolysis reaction. Besides, the process of the present invention is advantageous because high dilution reaction conditions are avoided, the reaction time is also reduced, the process and the work-up are simpler, it is also more economic and it is industrializable. Besides, the intermediates are isolated as solids. This fact is an additional advantage of the process of the invention.

Accordingly, an aspect of the present invention relates to a process for the preparation of entecavir of formula (I), or a pharmaceutically acceptable salt thereof, or a hydrate thereof, 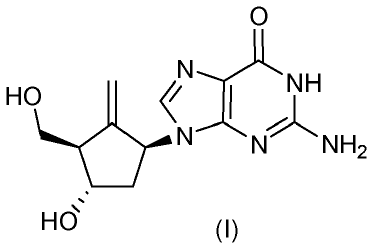 which comprises (a) submitting a compound of formula (III) where X is an halogen selected from Cl, Br, and I and R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro. 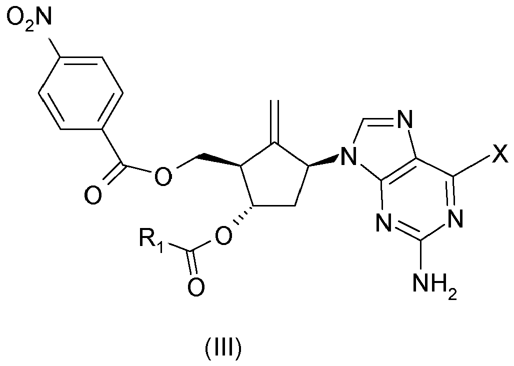 to a hydrolysis reaction using formic acid to yield a compound of formula (II) or a salt thereof, where R₁ has the same meaning as defined above; 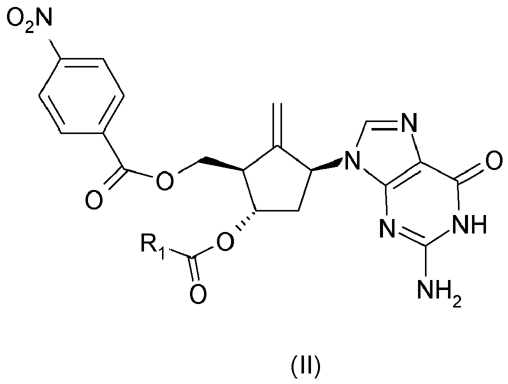 (b) submitting a compound of formula (II) to a deprotection reaction; and (c) isolating the entecavir either as free base or as a pharmaceutically acceptable salt.

Compound of formula (III) can be prepared by reacting a compound of formula (IV) where R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro, with a compound of formula (V) where X is selected from Cl, Br, and I.

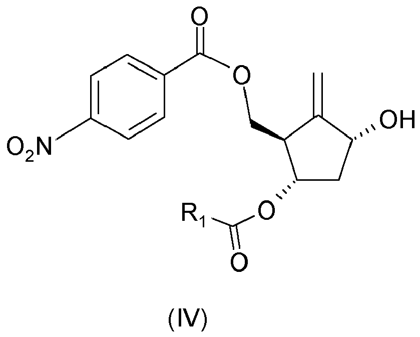

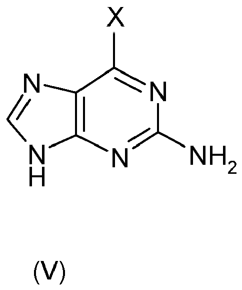

Compound of formula (IV) can be prepared by submitting a compound of formula (VI) where R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro, to a desilylation reaction to remove the TBS group.

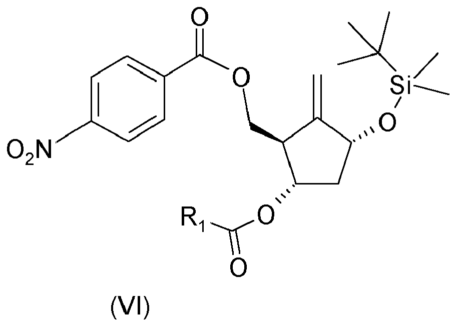

Compound of formula (VI) can be prepared by reacting a compound of formula (VII) where R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro, with a compound of formula (VIII) where Y is a halogen or with p-nitrobenzoyl anhydride.

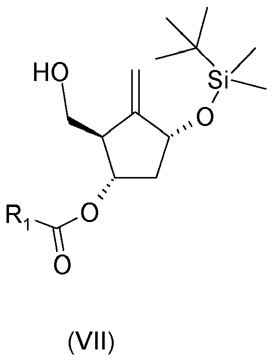

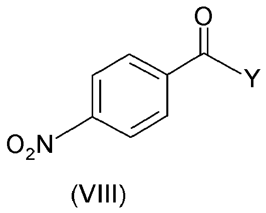

Several steps of the global process represents by themselves a significant improvement over the known prior art. Additionally, when different steps are carried out together the resulting process is particularly effective at industrial scale. Thus, it is also part of the present invention the provision of single reaction steps of the process comprising the sequence of steps described above from the starting compound of formula (VII) to the preparation of the entecavir (I), as well as the combination of two or more sequential steps of this process.

Several intermediate compounds which intervene in the process described above are new and form part of the invention. Thus, another aspect of the present invention is the provision of the compounds of formula (II) or its salts, (III) or its salts, (IV), and (VI).

Compounds (III), (IV), and (VI) have the advantage over other known compounds that are solid, thus, they can be isolated and purified easily. Compound (II) is also solid.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

In the formula of the compounds of the present invention, the use of bold and dashed lines to denote particular configuration of groups follows the IUPAC convention. A bond indicated by a broken line indicates that the group in question is below the general plane of the molecule as drawn (the "alpha" configuration), and a bond indicated by a bold line indicates that the group at the position in question is above the general plane of the molecule as drawn (the "beta" configuration).

The term "pharmaceutically acceptable salts" refers to salts prepared from pharmaceutically acceptable non-toxic bases or acids, including inorganic or organic bases and inorganic or organic acids. As entecavir is a basic compound, salts may be prepared form pharmaceutically acceptable non-toxic acids, including inorganic and organic acids. Such acids include for instance acetic, benzenesulfonic, benzoic, camphorsulfonic, citric, ethanesulfonic, fumaric, gluconic, glutamic, hydrobromic, hydrochloric, lactic, maleic, malic, mandelic, methanesulfonic, phosphoric, succinic, sulfuric, tartaric, p-toluensulfonic acid, and the like. Particularly preferred are citric, hydrobromic, hydrochloric, maleic, sulfuric, fumaric, and tartaric acids.

It will be understood that, as used herein, references to entecavir are meant to also include the pharmaceutically acceptable salts.

In the context of the invention, the term "selective reaction" refers to one that can occur with other substances or with different functional groups of the same substance but exhibits a degree of preference for the substance of interest or for the functional group of interest.

In the context of the invention, the term "stereoselective" refers to a given reaction that yields more of one stereoisomer of a set of stereoisomers.

By the term "room temperature" herein is understood a temperature of between 18-26 °C.

As mentioned above, it is provided a process for the preparation of entecavir which comprises several steps encompassing the transformation of a compound of formula (VII) to a compound of formula (I).

In a preferred embodiment of the process the compound of formula (VII) is that where R₁ is a (C₁-C₄)-alkyl. In a more preferred embodiment of the process the compound of formula (VII) is that where R₁ is methyl.

Additionally, compound of formula (VII) can be prepared from commercial starting materials, compound (XIV) and compound (XV), by a sequence of reactions which allow obtaining a chiral carbocyclic core having the hydroxyl groups differentiated, preferably by orthogonal protection with a TBS and an alkanoyl ester, preferably an acetyl (Ac) ester, a benzoyl ester, or a benzoyl ester substituted by a radical selected from the group consisting of (C₁-C₄)-alkyl, (C₁-C₄)-alkoxy, halogen, and nitro. This sequence of reactions includes the preparation of a specific stereoisomer of the compound of formula (XIII) by a highly stereoselective process which affords the desired stereoisomer of the free diol among the four possible stereoisomers. The selective monoprotection of the hydroxy of the propargylic position is achieved by the specific selection of the protective group TBS and the reaction conditions used. It also comprises a subsequent desilylation and epoxidation.

The following Scheme I illustrates a particular embodiment of the process for preparing the intermediate (VII) from commercial compounds.

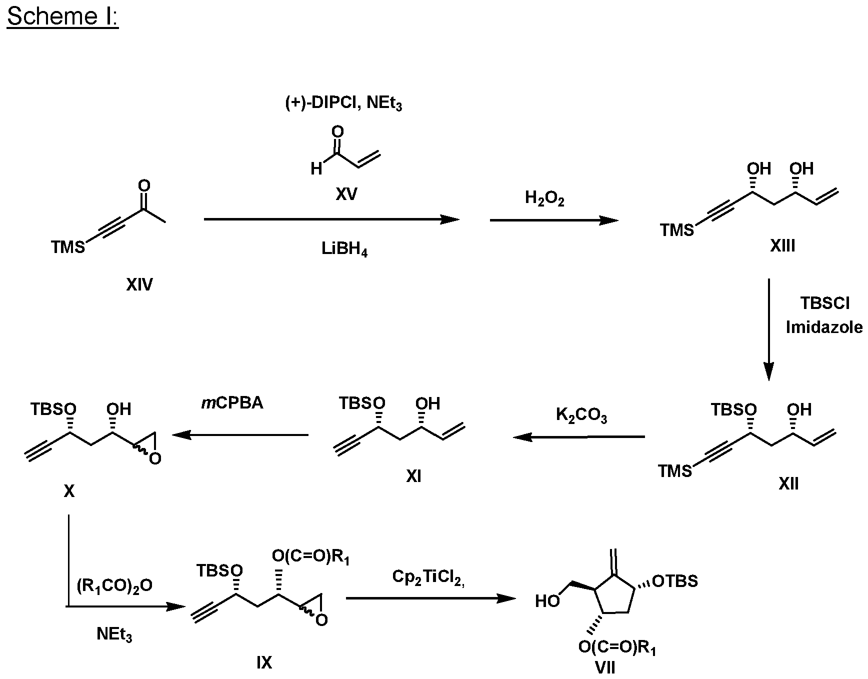

Hereinafter, a description of the preparation process by each step will be given in detail.

Compound of formula (XIII) is the following syn isomer: (3S,5R)-7-(trimethylsilyl)hept-1-en-6-yne-3,5-diol. It can be prepared from the compound of formula (XIV) and acrolein (XV), both commercially available. Thus, 4-trimethylsilyl-3-butyn-2-one of formula (XIV) can be reacted first with (+)-B-chlorodiisopinocampheylborane ((+)-DIPCI) and a tertiary amine in a suitable solvent, and then with acrolein of formula (XV), followed by submitting the compound thus obtained to a syn stereoselective reduction using a reducing agent such as lithium borohydride or sodium borohydride. The boron compound intermediate thus obtained is then treated with hydrogen peroxide, methanolamine, ethanolamine, or triethanolamine to cleave the boron moiety yielding to the compound of formula (XIII). Examples of tertiary amines include triethylamine, tributylamine, tripropylamine, N,N-diisopropylethylamine, or N,N,N',N'-tetramethylethane 1,2-diamine. Examples of suitable solvents used in the reaction between compound (XIV) and compound (XV) are a (C₃-C₆)-ether such as tetrahydrofuran, (C₁-C₆) chlorine containing solvents such as chloroform or dichloromethane, and their mixtures with (C₅-C₇) alkanes such as pentane, hexane or cyclohexane. The reaction is generally carried out at low temperatures, generally at -78 °C. Once the reaction is considered completed, it can be quenched for instance with a saturated solution of ammonium chloride. The final treatment of the boron compound intermediate can be carried out with H₂O₂. The solvent used can be a mixture of a (C₃-C₆)-ether and water, (C₁-C₄)-alcohol, and their mixtures with (C₅-C₇) alkanes such as pentane, hexane or cyclohexane.

Compound of formula (XIII) can be selectively monoprotected in the propargylic position by reacting it with tert-butyldimethylsilyl chloride (TBSCI) in the presence of imidazole and a suitable solvent to afford the compound of formula (XII). Examples of suitable solvents include (C₃-C₆)-ethers such as tetrahydrofuran and (C₁-C₇)-chlorine containing solvents such as dichloromethane.

The subsequent sequence of reactions comprises desilylation, epoxidation, and protection of the secondary hydroxyl to yield the compound of formula (IX), where R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

This sequence of steps yields to an oxiran pentynyl intermediate having two hydroxy groups orthogonally protected with different protective groups (TBS ether and a (C₁-C₆)-alkanoyl ester, benzoyl ester, or benzoyl ester substituted by a radical selected from the group consisting of (C₁-C₄)-alkyl, (C₁-C₄)-alkoxy, halogen, and nitro).

The desilylation reaction of the compound of formula (XII) can be carried out using an inorganic base such as an alkaline metal carbonate such as sodium carbonate or potassium carbonate or a alkaline hydroxide such as sodium hydroxide or potassium hydroxide, or diaza(1,3)bicyclo[5.4.0]undecane (DBU), in the presence of a solvent such as a (C₁-C₄)-alcohol, for instance, methanol, acetonitrile and mixtures of any of them with water.

The epoxidation reaction of the compound of formula (XI) can be carried out with an epoxidating agent in a suitable solvent. Examples of the epoxidating are m-chloroperbenzoic acid (m-CPBA), t-butyl hydroperoxide or cumenyl hydroperoxide. The epoxidation can be carried out in the presence of a catalyst. The catalyst can be a metal compound of vanadium, titanium or molybdenum. Examples of suitable solvents are (C₁-C₆)-chlorine containing solvents such as dichloromethane. Generally, this reaction is carried out at room temperature.

The esterification of the hydroxyl group of the compound of formula (X) previously obtained to yield the compound of formula (IX) can be carried out using acetic anhydride or acetyl chloride in the presence of a suitable solvent and a base. In such a case an acetyl ester is obtained. Other alkanoyl esters can be obtained using the corresponding alkanoic anhydride or alkanoyl halide. Examples of suitable solvents include (C₁-C₆)-chlorine containing solvents such as dichloromethane, (C₂-C₆)-ethers, (C₆-C₈)-aromatic hydrocarbons such as toluene, pyridine, or N,N-dimethylformamide. The esterification of the hydroxyl group as benzoyl or benzoyl substituted by the substituents mentioned above can be carried out from the corresponding benzoyl chloride or benzoic anhydride in the presence of a suitable solvent selected from those mentioned above.

Examples of suitable bases include triethylamine, tributylamine or tripropylamine, triethylamine/4-dimethylaminopyridine, diisopropylethylamine, pyridine, 4-dimethylaminopyridine, collidine or imidazole.

Compound of formula (IX) can be submitted to a catalytic radicalary cyclization reaction using Cp₂TiCl₂ as catalyst to yield the compound of formula (VII) where R₁ has the same meanings as those mentioned above for compound (IX). A mixture of Zn, 2,4,6-collidine and TMSCI can be used to recover the catalyst. A mixture of Mn and 2,4,6-collidine hydrochloride can also be used to carry out the cyclization of the compound of formula (IX). Generally the solvent used is an (C₃-C₆)-ether such as tetrahydrofuran or 2-methyl tetrahydrofuran.

Compound (VII) can be submitted to an esterification reaction in order to protect the free OH group as nitrobenzoyl group, yielding the compound of formula (VI). This reaction can be carried out with a p-nitrobenzoyl halide, preferably a p-nitrobenzoyl chloride, or p-nitrobenzoyl anhydride, generally, in the presence of a base and an appropriate solvent. Examples of bases include (C₃-C₈)-organic amines such as triethylamine, diisopropylamine, diisopropylethylamine, pyridine or imidazole. Preferably, the organic base is a tertiary amine. More preferably, the tertiary amine is triethylamine. If desired, a catalytic amount of 4-dimethylaminopyridine can be used to accelerate the reaction. Examples of solvents include (C₁-C₆)-chlorine containing solvents such as dichloromethane, chloroform, chlorobenzene; (C₂-C₈)-ethers such as tetrahydrofuran, diethyl ether or dioxane; and (C₆-C₈)-aromatic hydrocarbons such as toluene or xylene. Generally the reaction is carried out at a temperature of between 0 °C and room temperature.

In a preferred embodiment of the process, compounds of formula (VII), (VI), (IV), (III), and (II) are those where R₁ is a (C₁-C₄)-alkyl. In a more preferred embodiment of the process, compounds of formula (VII), (VI), (IV), (III), and (II) are those where R₁ is methyl.

The compound of formula (VI) is a key intermediate in the preparation of entecavir.

The desilylation reaction over the compound of formula (VI) to remove the TBS group yields to the compound of formula (IV). This reaction can be carried out by acid catalysis or with fluorides. Examples of appropriate acids are camphorsulfonic acid (CSA), benzenesulfonic acid, p-toluensulfonic acid (TsOH), methanesulfonic acid (MsOH), pyridinium p-toluensulfonate (PPTS), or acetic acid. Examples of appropriate fluoride compounds are fluorhydric acid or tetra-n-butyl ammonium fluoride (TBAF). Generally the reaction is carried out in a suitable solvent for instance, methanol, tetrahydrofuran, pyridine, and a mixture thereof. In a preferred embodiment, a sulphonic acid and an alcohol as a solvent is used. In a still more preferred embodiment, the acid is selected from the group consisting of benzenesulfonic acid, toluensulfonic acid, methanesulfonic acid, pyridinium p-toluensulfonate and the solvent is methanol. In another preferred embodiment, a fluoride and an ether as a solvent is used. In another still more preferred embodiment, the fluoride is selected from fluorhydric acid and tetra-n-butyl ammonium fluoride and the solvent is tetrahydrofuran.

The coupling between the compound of formula (IV) and the 2-amino-6-halopurine of formula (V) to yield the compound of formula (III) can be carried out in the presence of a phosphine, an appropriate solvent and an azodicarboxylate. Preferably, the phosphine is selected from the group consisting of triphenylphosphine (PPh₃), tributylphosphine (Bu₃P), 1,2-Bis(diphenylphosphino)ethane (dppe), and diphenyl(2-pyridyl)phosphine. More preferably, the phosphine is triphenylphosphine or tributylphosphine. Also preferably, the azodicarboxylate is selected from the group consisting of diethylazodicarboxylate (DEAD), diisopropylazodicarboxylate (DIAD). Examples of solvents include (C₁-C₆)-chlorine containing solvents such as dichloromethane, chloroform, chlorobenzene; (C₂-C₈)-ethers such as tetrahydrofuran, diethyl ether or dioxane; (C₆-C₈)-aromatic hydrocarbons such as toluene or xylene; N,N-dimethylformamide, acetonitrile, or mixtures thereof. In a preferred embodiment, the solvent is tetrahydrofuran.

The reaction is carried out at a temperature of between -40 °C and 40 °C. preferably between -10 °C and 20 °C.

In a preferred embodiment of the process, the compound of formula (V) is that where X is Cl.

The hydrolysis of the halopurine (III) to yield the compound of formula (II) is carried out with formic acid. The use of this acid contribute to the selectivity of the reaction. In a preferred embodiment, the hydrolysis reaction is carried out with aqueous formic acid. In a particular embodiment, the formic acid is aqueous formic acid 80% w/w. Preferably, the the hydrolysis of the halopurine is carried out at a temperature of between 20 and 90 °C. More preferably, the hydrolysis of the halopurine is carried out at a temperature of between 40 °C and 80 °C. In a more preferred embodiment, the halopurine (III) is that where X is Cl.

The reaction conditions used allows precipitating the compound of formula (II) from water. The compound of formula (II) can be isolated directly from the reaction medium as a free base or as a salt thereof, for example as hydrohalide, preferably as hydrochloride, or as a formiate. Generally, the isolated product is obtained with high yield and purity, the purity being higher than 85% a/a by HPLC.

The deprotection reaction of the compound of formula (II) thus obtained to yield entecavir can be carried out with a base and an appropriate solvent. Preferably, the base is selected from the group consisting of a metal alkaline (C₁-C₄)-alkoxide such as sodium methoxide, a metal alkaline hydroxide such as sodium or potassium hydroxide, a metal alkaline carbonate such as sodium or potassium carbonate, and 1,8-diazabicyclo[5.4.0]undec-7-ene (DBU). A mixture of KCN/EtOH or a mixture of NH₃/MeOH can also be used.

In a preferred embodiment, the deprotection is carried out using a metal alkaline (C₁-C₄)-alkoxide as base and a (C₁-C₄)-alcohol as solvent. Preferably, the base is sodium methoxide and the solvent is methanol.

The deprotection reaction conditions used are mild and allow obtaining entecavir from the compound of formula (II) with purity higher than 97% a/a by HPLC and higher than 99% after its recrystallization.

The most adequate conditions for carrying out the process vary depending on the parameters considered by an expert in the art, such as the concentration, the temperature, the solvent used during the reaction or the isolation of the products, and the like. These can be readily determined by said skilled person in the art with the help of the teachings given above and the examples given in this description.

The entecavir obtained by the process of the invention can be isolated from the reaction medium as a free base or as a pharmaceutically acceptable salt after routinely work-up and/or crystallization.

The pharmaceutically acceptable salts can also be obtained from the entecavir base by treatment with the corresponding pharmaceutically acceptable acid. It can also be obtained by treatment with the corresponding pharmaceutically acceptable base. Alternatively, a pharmaceutically acceptable salt of the entecavir obtainable according to the process of the invention is converted into another pharmaceutically acceptable salt. Entecavir, including their salts can exist in solvated, as well as unsolvated forms, including hydrated forms. Thus, they can contain in their structure stoichiometric amounts of solvent in the case of solvates, or of water in the case of hydrates. It is to be understood that the process of the invention encompasses the preparation of all such solvated, as well as unsolvated forms. The obtention of solvates and hydrates depends on the solvent used and the crystallization conditions that can be determined by the skilled person.

In a preferred embodiment of the process, it is obtained entecavir in the form of a monohydrate (water content of 6.5-6.7% as determined by Karl Fisher). Thus, entecavir can be directly precipitated from the reaction media where the solvent is water, obtaining entecavir monohydrate with a yield of around 70 %. This is an advantage of this process since with other approaches different impurities are generated (dimmers, elimination products, etc) which do not allow isolating the entecavir by precipitation. The Examples illustrate these facts.

As mentioned above several intermediates compounds of this preparation process are new and form part of the invention. Thus, compounds of formula (II) or its salts, (III) or its salts, (IV), and (VI) are also part of the invention.

Preferably, the compounds of formula (II), (III), (IV) and (VI) are those where R₁ is a (C₁-C₄)-alkyl. More preferably, the compounds of formula (II), (III), (IV) and (VI) are those where R₁ is methyl.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1: Preparation of (3S,5R)-7-(trimethylsilyl)hept-1-en-6-yne-3,5-diol (XIII)

To a solution of (+)-DIPCI (90-105 %) (25 g, 77.94 mmol) in anhydrous tetrahydrofuran (THF) (40 mL) at 0 °C under N₂ atmosphere, triethylamine (NEt₃) (85.02 mmol, 11.6 mL) was added under stirring. Then, 4-trimethylsilyl-3-butyn-2-one (XIV) 98% (70.85 mmol, 9.78 g) was added dropwise and the reaction mixture was stirred 2 h at - 5 °C - 0 °C. Then the solution was cooled to - 78 °C. A solution of acrolein (90% purity) (102.6 mmol, 7.62 mL) in anhydrous THF (20 mL) was added slowly and the reaction mixture was stirred 1 h at - 78 °C. A solution of lithium borohydride (LiBH₄) 2 M in hexane (106.28 mmol, 53 mL) was added slowly. The reaction mixture was further stirred 1 h at - 78 °C, and then quenched carefully with saturated solution of NH₄Cl (40 mL) for 0.5 h. (temperature increased from - 78 °C to room temperature). The mixture was partitioned with H₂O (40 mL) and tert-butyl methyl ether (TBME) (90 mL). Water layer was extracted with TBME (25 mL). The organic phases were combined, dried (MgSO₄) and the volatiles were removed in vacuo. The residue was yellow pale oil (62 g).

To the residue, a THF:H₂O 3:1 (80 mL) was added at room temperature under N₂ atmosphere. Then, solid AcONa (53.64 mmol, 4.40 g) was added in one portion and the mixture was cooled to 0 °C. Then H₂O₂ 30% (30 mL, 5 equivalents) was added dropwise during 10 min and the mixture was stirred further 10 min at 0 °C and 30 min at room temperature. Then the mixture was cooled to 0 °C and a saturated solution of Na₂S₂O₃ (30 mL) was added slowly. The reaction mixture was stirred further 10 min at 0 °C and 15 min at room temperature. Then a mixture of H₂O (20 mL) and TBME (35 mL) was added. The organic layer was poured of. Water layer was extracted with TBME (10 mL). The organic phases were combined, dried (MgSO₄) and the volatiles were removed in vacuo. Then the resulting oily substance (49.2 g) was isolated by flash chromatography on silica gel (Hexane-ethyl acetate from 90:10 to 60:40) to give 9.13 g of a mixture of diols with an enantiomeric ratio (e.r). 80:20 and Sin/Anti ratio 90:10 (yield 66%). Recrystallization in hexanes affords the title compound as a white crystalline solid (5.2 g, 57% overall yield, e.e. 96%). Mp (°C): 80 - 82. Rf (hexane:AcOEt 80:20) = 0.3. [ α]_{D} = + 2.3 (c 1.0, CHCl₃). IR (Film) (cm⁻¹): 3349, 2956, 2176. EM (+ESI high resolution): m/z 221.0969 [M+Na]⁺.

### Example 2: Preparation of (3S,5R)-5-(tert-butyldimethylsilyloxy)-7-(trimethylsilyl)hept-1-en-6-yn-3-ol (XII)

To a solution of diol (XIII) (5.0 g, 25.2 mmol) and imidazole (2.1 g, 30.3 mmol) in anhydrous THF (60 mL) at 0 °C under N₂ atmosphere, a solution of TBSCI (4.23 g, 27.7 mmol) in anhydrous THF (20 mL) was added dropwise and the reaction mixture was warmed up to room temperature. The reaction mixture was stirred for 5 h. Then, a 22% solution of NH₄Cl (25 mL) was added slowly and the reaction mixture was stirred 10 min. The mixture was partitioned and the organic phase was dried (MgSO₄), and the volatiles were removed in vacuo. Then the resulting oily substance was purified by flash chromatography on silica gel (hexane-ethyl acetate from 90:10 to 80:20) to give 4.84 g (61 %) of the compound of the title. Light yellow oil. Rf (hexane:AcOEt 80:20) = 0.55. [α]_{D} = + 39.9 (c 1.0, CHCl₃). IR (Film) (cm⁻¹): 3424, 3081, 2958, 2172.

### Example 3: Preparation of (3S,5R)-5-(tert-butyldimethylsilyloxy)hept-1-en-6-yn-3-ol (XI)

K₂CO₃ (0.101 g, 0.73 mmol) was added in one portion to a stirred solution of (XII) (0.455 g, 1.46 mmol) in anhydrous methanol (MeOH) (4.5 mL) at room temperature under N₂ atmosphere. The reaction mixture was stirred for 1 h. After completion of the reaction, the volatiles were removed and CH₂Cl₂ (10 mL) was added to the residue. The mixture was filtered, dried (MgSO₄) and the volatiles were removed to give 0.366 g (yield: 100%) of compound of the title. Light yellow oil. Rf (hexane:AcOEt 80:20) = 0.43. [α]_{D} = + 32.7 (c 1.0, CHCl₃). IR (Film) (cm⁻¹): 3417, 3079, 2956, 2109.

### Example 4: Preparation of (1S,3R)-3-(tert-butyldimethylsilyloxy)-1-(oxiran-2-yl)pent-4-yn-1-ol (X)

A suspension solution of *m*CPBA 77% (13.64 g, 60.83 mmol) in anhydrous CH₂Cl₂ (30 mL) at room temperature under N₂ atmosphere was added to a stirred solution of (XI) (5.85 g, 24.33 mmol) in anhydrous CH₂Cl₂ (20 mL). The reaction mixture was stirred at room temperature for several hours. After completion of the reaction, the reaction mixture was filtered and washed with saturated Na₂S₂O₃ solution (35 mL), saturated NaHCO₃ (2 x 15 mL). The combined organic phases were dried (MgSO₄) and the volatiles were removed to give the compound of the title (5.53 g, yield: 89%) as yellow oil. ¹H NMR (400 MHz, CDCl₃) δ 4.56 (tt, J = 6.65, 6.65, 2.21, 2.21 Hz, 1H), 3.86 (td, J = 8.17, 3.98, 3.98 Hz, 1H), 3.72-3.65 (m, 1H), 2.95 (ddd, J = 8.03, 4.10, 1.96 Hz, 1H), 2.71 (td, J = 5.09, 3.45, 3.45 Hz, 1H), 2.69-2.63 (m, 1H), 2.38 (dd, J = 7.67, 2.10 Hz, 1H), 1.98-1.81 (m, 1H), 0.81 (s, 1H), 0.08 (s, 1H), 0.08 (s, 1H), 0.06 (s, 1H), 0.05 (s, 1H). ¹³C NMR (101 MHz, CDCl₃) δ 84.3, 73.4, 73.4, 69.5, 68.0, 61.5, 61.1, 55.0, 54.1, 44.7, 44.2, 42.2, 41.7, 25.6, 18.0, -4.4, -5.1.

### Example 5: Preparation of (1S,3R)-3-(tert-butyldimethylsilyloxy)-1-(oxiran-2-yl)pent-4-ynyl acetate (IX with R₁= methyl, IXa)

To a solution of (X) (5.5 g, 21.45 mmol) and catalytic amount of DMAP in anhydrous CH₂Cl₂ (50 mL) at 0 °C under N₂ atmosphere, NEt₃ (3.8 mL, 27.89 mmol) was added dropwise. Then, Ac₂O (2.4 mL, 25.74 mmol) was added thereto dropwise at 0 - 5 °C. The reaction mixture was warmed to room temperature and stirred for 1 h. Then, a saturated NH₄Cl solution (35 mL) was added slowly and the reaction mixture was stirred 10 min. The mixture was partitioned and the aqueous phase was extracted with CH₂Cl₂ (20 mL). The organic phases were combined and washed with saturated NaHCO₃ solution (30 mL). The basic aqueous phase was extracted with CH₂Cl₂ (20 mL). The organic phases were combined, dried (MgSO₄), and the volatiles were removed in vacuo to afford the compound of the title as a yellow pale oil (6.39 g, yield: 99%). ¹H NMR (400 MHz, *CDCl₃)* δ 5.07-4.99 (m, 1H), 4.54-4.48 (m, 1H), 3.16 (ddd, *J* = 5.71, 4.12, 2.63 Hz, 1H), 3.05 (ddd, *J* = 4.87, 3.88, 2.70 Hz, 1H), 2.82 (dd, *J* = 4.81, 4.18 Hz, 1H), 2.77-2.70 (m, 1H), 2.67 (dd, *J* = 4.84, 2.63 Hz, 1H), 2.44 (d, *J* = 2.14 Hz, 1H), 2.43 (d, *J* = 2.14 Hz, 1H), 2.08 (s, 1H), 2.06 (s, 1H), 2.16-1.97 (m, 1H), 0.90 (s, 1H), 0.15 (s, 1H), 0.14 (s, 1H), 0.12 (s, 1H), 0.12 (s, 1H). ¹³C NMR (101 MHz, *CDCl₃)* δ169.9, 84.0, 83.9, 73.3, 73.2, 70.9, 69.8, 59.6, 52.9, 52.1, 45.1, 39.8, 39.6, 25.6, 20.9, 20.9, 18.0, -4.5, -5.1.

### Example 6: Preparation of (1S,2R,4R)-4-(tert-butyldimethylsilyloxy)-2-(hydroxymethyl)-3-methylenecyclopentyl acetate (VII with R₁= methyl, VIIa)

Strictly deoxygenated anhydrous THF (15 mL) was added to a mixture of C_{P2}TiCl₂ (0.166 g, 0.67 mmol) and activated Zn powder (0.65 g, 10.05 mmol) under N₂ atmosphere and the suspension was stirred at room temperature until it turned lime green. Then, a solution of epoxide (IX) (1.0 g, 3.35 mmol) and dry 2,4,6-collidine (2.6 mL, 20.01 mmol) in strictly deoxygenated anhydrous THF (18.5 mL) was added. Then, TMSCI (1.3 mL, 10.05 mmol) was added slowly. The reaction mixture was stirred for 4 h and filtered through Celite®. The diatomaceous earth was washed with TBME (20 mL). The organic phases were combined, washed with 2 M HCl (2 x 10 mL), H₂O (10 mL) saturated NaHCO₃ solution (5 mL) and dried (MgSO₄). The volatiles was removed and the resulting oily substance (0.98 g) was isolated by flash chromatography on silica gel (Hexane-ethyl acetate from 90:10 to 60:40) to give 0.40 g (41 % yield) of the compound of the title. Light yellow oil. [α]_{D} = - 43.5 (c 1.0, CHCl₃). IR (Film) (cm⁻¹): 3447, 3085, 2955, 1734. Ratio cis:trans 5:95. The subproduct of reductive elimination is in an amount less than 5% w/w.

### Example 7: Preparation of (1S,2R,4R)-4-(tert-butyldimethylsilyloxy)-2-(hydroxymethyl)-3-methylenecyclopentyl acetate (VII with R₁= methyl, VIIa)

Strictly deoxygenated anhydrous THF (15 mL) was added to a mixture of IrCl(CO)PPh₃)₂ (0.26 g, 0.34 mmol) and manganese powder (0.55 g, 10.05 mmol).Then, a solution of 2,4,6-collidine (3.5 mL, 26.8 mmol) and (1 S,3R)-3-(tert-butyldimethylsilyloxy)-1-(oxiran-2-yl)pent-4-ynyl acetate (IX) (1.0 g, 3.35 mmol) in strictly anhydrous THF (22 mL) was added at room temperature. Then, TMSCI (1.7 mL, 13.4 mmol) was added. Finally, a solution of Cp₂TiCl₂ (0.167 g, 0.67 mmol) in strictly deoxygenated anhydrous THF (12 mL) was added. The reaction mixture was stirred for 4 h under H₂ atmosphere at room temperature. Water (5 mL) was added and the mixture was stirred for 10 min and filtered through Celite®. The diatomaceous earth was washed with TBME. The organic phases were combined and acidified with HCl until pH 2. The mixture was stirred 15 min and the phases were separated. The organic phase was washed with H₂O and dried over Na₂SO₄. The volatiles were removed to obtain 1.383 g of an oily substance (estimated yield by RMN-¹H 58 %).

### Example 8: Preparation of ((1R,3R,5S)-5-acetoxy-3-(tert-butyldimethylsilyloxy)-2-methylenecyclopentyl)methyl 4-nitrobenzoate (VI with R₁= methyl)

To a solution of (1 S,2R,4R)-4-(tert-butyldimethylsilyloxy)-2-(hydroxymethyl)-3-methylenecyclopentyl acetate (VII with R₁= methyl) (103.50 g, 344.46 mmol) and catalytic amount of DMAP in anhydrous CH₂Cl₂ (518 ml) at 0 °C under N₂ atmosphere, NEt₃ (72.02 ml, 516.69 mol) was added dropwise. Then, a solution of p-nitrobenzoyl chloride (76.70g, 413.35 mmol) in anhydrous CH₂Cl₂ (103.50 ml) was added thereto dropwise at 0-5 °C. The reaction mixture was warmed to room temperature and stirred for 2 h. Then, a saturated solution of NH₄Cl (621 ml) was added slowly and the reaction mixture was stirred 15 min. The mixture was partitioned and the organic phase was washed with H₂O adjusting the pH at 7. The organic phases were combined and the volatiles were removed in vacuo to obtain 185.12 g of an oil. This product was crystallized in MeOH to afford the compound of the title as a white crystalline solid (92.74g, 60% yield, 94% HPLC purity).¹H NMR (400 MHz, cdcl₃) δ 8.31 - 8.13 (4H, m), 5.27 (1H, t, *J* = 2.3 Hz), 5.13 (1H, t, *J* = 2.3 Hz), 5.05 (1H, dt, *J* = 8.4, 6.6 Hz), 4.48 (3H, m), 3.18 - 3.08 (1H, m), 2.59 - 2.44 (1H, m), 1.99 (3H, s), 1.79 - 1.64 (1H, m), 0.91 (9H, s), 0.10 (3H, s), 0.09 (3H, s). ¹³C NMR (101 MHz, cdcl₃) δ 170.87, 164.59, 150.75, 150.54, 135.53, 130.78, 123.73, 110.09, 73.21, 72.55, 66.31, 46.90, 40.55, 25.92, 21.18, 18.31, -4.51, -4.65.

### Example 9: Preparation of ((1R,3R,5S)-5-acetoxy-3-hydroxy-2-methylenecyclopentyl)methyl 4-nitrobenzoate (IV with R₁= methyl)

To a solution of ((1R,3R,5S)-5-acetoxy-3-(tert-butyldimethylsilyloxy)-2-methylenecyclopentyl)methyl 4-nitrobenzoate (VI with R₁= methyl) (12.17g, 27.07mmol) in anhydrous MeOH (109.5 ml) at room temperature under N₂ atmosphere, a solution of (+)-CSA (0.314g, 1.35 mmol) in anhydrous MeOH (6.1 ml) was added dropwise. The reaction mixture was stirred for 3 h and then was cooled to 0°C and stirred for 1 h. After completion of the reaction, water (49 ml) was added and the solution was neutralized. Then, MeOH was removed in vacuo and the water layer was extracted with TBME. The organic phases were combined and the volatiles were removed in vacuo. The resulting oily substance (11.42 g) was crystallized in a mixture of TBME: heptane 1:4 to afford the compound of the title as a white crystalline solid (7.63 g, 84% yield, 84% HPLC purity).¹H NMR (400 MHz, cdcl₃) δ 8.24 (4H, td, *J* = 8.8, 4.4 Hz), 5.44 (1H, bs), 5.25 (1H, d, *J* = 1.5 Hz), 5.17 (1H, q, *J* = 5.5 Hz), 4.55 (1H, d, *J* = 5.1 Hz), 4.52 - 4.43 (1H, m), 4.42 - 4.30 (1H, m), 3.20 (1H, d, *J* = 4.5 Hz), 2.51 (1H, dt, *J* = 13.0, 6.5 Hz), 2.02 (3H, s), 1.85 (1H, dt, *J* = 12.4, 5.7 Hz). ¹³C NMR (101 MHz, cdcl₃) δ 170.58, 164.64, 151.28, 150.77, 135.40, 130.85, 123.77, 112.34, 74.87, 73.24, 65.87, 47.93, 40.07, 21.26.

### Example 10: Preparation of ((1R,3S,5S)-5-acetoxy-3-(2-amino-6-chloro-9H-purin-9-yl)-2-methylenecyclopentyl)methyl 4-nitrobenzoate (III with R₁= methyl)

A solution of 2-amino-6-chloropurine (7.38 g, 43.54 mmol) and triphenylphospine (11.40 g, 43.54 mmol) in anhydrous THF (927 mL) at room temperature under N₂ atmosphere was stirred 15 min. The mixture was cooled to - 10 °C, and DIAD (8.60 mL, 43.54 mmol) was added dropwise and stirred for 10 min. Then, a solution of ((1R,3R,5S)-5-acetoxy-3-hydroxy-2-methylenecyclopentyl)methyl 4-nitrobenzoate (IV with R₁= methyl) (7.30 g, 21.77 mmol) in anhydrous THF (160 mL) was added in 1 h. The reaction mixture was stirring for 3 h at - 10 °C. Then, the reaction mixture warmed up, filtered and the residue was washed with THF (109 mL). The solvent was partially distilled and then, isopropanol (440 mL) was added. The solvent was again partially distilled and the resulting solution was stirred and filtered to afford 6.51 g (61 % Yield, 95% HPLC purity) of title compound as a yellow pale solid.¹H NMR (400 MHz, cdcl₃) δ 8.28 (4H, dd, *J* = 20.6, 8.9 Hz), 7.80 (1H, s), 5.58 - 5.40 (2H, m), 5.33 (1H, bs), 4.91 (1H, s), 4.85 (1H, dd, *J* = 11.4, 9.3 Hz), 4.62 (1H, dd, *J* = 11.4, 6.3 Hz), 3.26 (1H, t, *J* = 7.3 Hz), 2.85 (1H, ddd, *J* = 14.3, 10.6, 5.2 Hz), 2.42 (1H, dd, *J* = 14.1, 8.1 Hz), 2.08 (s, 1H). ¹³C NMR (101 MHz, cdcl₃) δ 170.32, 164.96, 158.88, 153.13, 151.78, 150.84, 146.24, 141.90, 135.20, 130.94, 125.91, 123.85, 113.59, 74.57, 65.93, 57.37, 48.69, 35.52, 25.48, 21.27.

### Example 11: Preparation of ((1R,3S,5S)-5-acetoxy-3-(2-amino-6-oxo-1H-purin-9(6H)-yl)-2-methylenecyclopentyl)methyl 4-nitrobenzoate (II with R₁= methyl)

A solution of ((1R,3S,5S)-5-acetoxy-3-(2-amino-6-chloro-9H-purin-9-yl)-2-methylenecyclopentyl)methyl 4-nitrobenzoate (III with R₁= methyl) (6.30 g, 12.94 mmol) and formic acid 80% (126 mL) at 50 °C under N₂ atmosphere was stirred 9 h. Then, the solvent was removed in vacuo. H₂O (72 mL) was added and the suspension was stirred several hours at room temperature. Then, the suspension was filtered and the isolated solid was dried to afford 5.59 g (92% yield, 84.6% HPLC purity) of title compound as a yellow solid. ¹H NMR (400 MHz, dmso) δ 11.08 (1H, bs), 8.37 (2H, d, *J* = 8.8 Hz), 8.24 (2H, d, *J* = 8.8 Hz), 7.74 (1H, s), 6.66 (2H, s), 5.42 - 5.35 (1H, m), 5.35 - 5.31 (1H, m), 5.27 (1H, bs), 4.67 (1H, bs), 4.57 (2H, d, *J* = 7.4 Hz), 3.17 - 3.10 (1H, m), 2.70 (1H, ddd, *J* = 13.6, 11.3, 5.3 Hz), 2.34 - 2.25 (1H, m), 2.01 (3H, s). ¹³C NMR (101 MHz, dmso) δ 169.88, 166.77, 164.29, 157.13, 153.95, 151.30, 150.33, 147.92, 135.86, 135.08, 130.71, 123.92, 116.53, 111.19, 74.15, 65.60, 55.13, 47.81, 35.24, 20.96.

### Example 12: Preparation of 2-amino-9-((1S,3R,4S)-4-hydroxy-3-(hydroxymethyl)-2-methylenecyclopentyl)-1H-purin-6(9H)-one monohydrate (I)

To a solution of ((1R,3S,5S)-5-acetoxy-3-(2-amino-6-oxo-1H-purin-9(6H)-yl)-2-methylenecyclopentyl)methyl 4-nitrobenzoate (II with R₁= methyl) (5.20 g, 11.10 mmol) in anhydrous MeOH at room temperature under N₂ atmosphere, a solution MeONa 30% (4.1 mL, 22.20 mmol) was added dropwise. The reaction mixture was stirred for 30 min at room temperature and cooled to 0 °C. TBME (52 mL) was added and the mixture was neutralized with HCl to pH = 7. The phases were separated and the aqueous layer was extracted with TBME. Then, the aqueous phase was distilled until 45 mL. The suspension was heated at 85 °C and the clear suspension was slowly cooled to room temperature and stirred about 15 h. Then the suspension was filtered and the isolated solid was dried under vacuum to afford 2.37 g (72% yield, 98.8 % HPLC purity) of the title compound as a white solid (KF = 6.5%).The white solid was recrystallized in water to afford 64 % overall yield, 99.47 % HPLC purity (KF = 6.7%). ¹H NMR (400 MHz, dmso) δ 10.56 (1H, bs), 7.66 (1H, s), 6.41 (2H, bs), 5.36 (1H, dd, *J* = 10.4, 7.9 Hz), 5.10 (1H, bs), 4.87 (1H, d, *J* = 3.1 Hz), 4.83 (1H, t, *J* = 5.3 Hz), 4.56 (1H, bs), 4.30 - 4.14 (1H, m), 3.53 (2H, t, *J* = 6.0 Hz), 2.56 - 2.51 (1H, m), 2.27 - 2.15 (1H, m) 2.10 - 1.98 (1H, m). ¹³C NMR (101 MHz, dmso) δ 166.75, 158.13, 154.66, 151.56, 151.43, 135.57, 116.24, 109.16, 70.40, 63.04, 55.06, 54.09.

### Comparative Example 1: Influence of the order in which the steps of hydrolysis and deprotection are carried out

When it was first carried out the deprotection of the ester groups of the compound of formula (III) and then the hydrolysis of the deprotected halopurine obtained, it was observed that the formation of impurities was drastically increased and the yield of the reaction was very low.

The deprotection reaction with MeONa /MeOH yields to a solid 70:30 mixture of 6-chloroentecavir and 6-methoxyentecavir with quite high yield. However, the treatment with formic acid of the previous mixture to effect the hydrolysis led to the formation of two major impurities (15 and 8% area percentage by HPLC) corresponding to the by-products resulting from the formylation of each of the free hydroxyl groups. The yield of the reaction was very low (22%), and it was not increased by increasing the reaction time.

The HPLC conditions used are the following: Column: ZORBAX Eclipse XDB-C18 150x4.6 mm, 5µm; mobile phase: A (water) and B (ACN); gradient A/B at t (min)= 0 80/20 and at t(min) = 20 20/80; detection: 254 nm; flow: 0.6 mL/min; injection: 5 µL.

### Comparative Example 2: Effect of the acid in the hydrolysis of the halopurine (III) to yield compound of formula (II)

This Example illustrates the effect of the combination of the specific protective groups with the use of formic acid. The starting material is the compound of formula (III) with X= Cl.

The following Table 1 shows the reaction conditions and the results obtained.

**Table 1:**

| Examples | Acid | t (h) | T (°C) | Total impurities (area % by HPLC) | (I) (area % by HPLC) | (II) (area % by HPLC) | Isolation of (II) |
|---|---|---|---|---|---|---|---|
| 2a | HCOOH | 2 | 70 | 12% | 0 | 85% | Yes, Solid Quantitative yield |
| Comparative Example 2b | HCl 1M | 2 | 70 | 0 | 0 | 0 | No reaction |
| Comparative Example 2c | HCl 2.5M | 1 | 75 | 43% | 51% | 0 | No isolable |

As it can be seen in the previous table good results are obtained using formic acid. On the contrary, the reaction does not occur with HCl 1 M and with HCl 2.5 M the hydrolysis was not selective and deprotection also took place, thus yielding Entecavir (I) with important amount of impurities.

### Comparative Example 3: Effect of the protective groups in the hydrolysis of the halopurine

These results illustrate that the specific combination of protective groups used in the process of the present invention provides a special resistance in front of partial deprotection of the hydroxyl groups, which allows obtaining a high selectivity during hydrolysis of the halo group into the oxo group.

The starting material for the Example of the invention, Example 3a, is the compound of formula (II).

The comparative Examples have been carried out from a compound of formula (A).

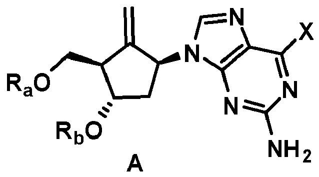

The following Table 2 illustrates the reaction conditions and the results obtained.

**Table 2:**

| Examples | Starting material A | Total Major impurities | Final product |
|---|---|---|---|
| | Rₐ/R_{b} | (area % by HPLC) | (area % by HPLC) |
| 3a (equal to Example 2a) | (III) | 12% | 85% (II) |
| Comparative Example 3b | Rₐ=R_{b}=Acetyl | 23% | 47% |
| | X=Cl | | (B where Rₐ=R_{b}=Acetyl) |
| Comparative Example 3c | Rₐ=R_{b}=H | 63% | 22% (I) |
| | X=Cl/OMe 70:30 mixture | | |

The compound B with Rₐ=R_{b}=Ac refers to entecavir diacetylated.

When the starting material is a compound A where Rₐ = R_{b} = acetyl and X= Cl, the reaction takes place with a low selectivity yielding important amounts of products resulting from the partial deprotection of the acetyl groups. On the other hand, when the starting material is a compound A where Rₐ=R_{b}=H important quantities of formylated byproducts are obtained. Moreover, when the starting material is a compound A where X=OMe, no hydrolysis of the methoxy group into the oxo group was observed by formic acid treatment.

### REFERENCES CITED IN THE APPLICATION

- EP481754-A
- CN1566118-A
- C N 1861602-A
- WO2004/52310-A
- W02010/74534-A
- CN101130542A
- CN101148450A
- WO2011/46303
- Li Wei Guo et al., "A new route for synthesis of Entecavir", Chinese Chemical Letters 2006 vol. 17, pp. 907-910.

## Claims

1. A process for the preparation of entecavir of formula (I), or a pharmaceutically acceptable salt thereof, or a hydrate thereof, 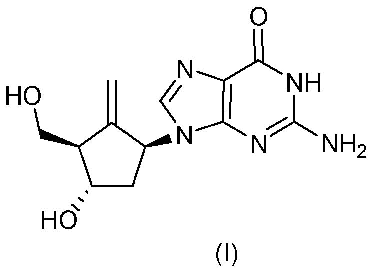 which comprises:
(a) submitting a compound of formula (III) wherein X is an halogen selected from Cl, Br, and I, and R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro. 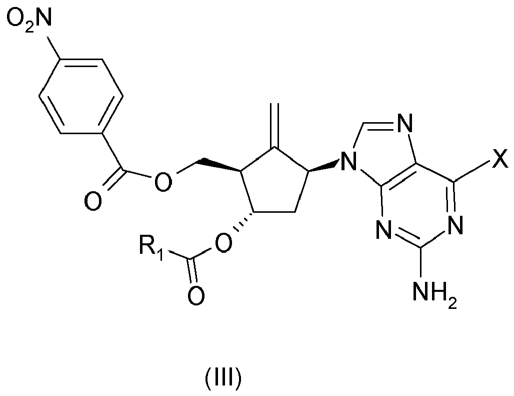 to a hydrolysis reaction using formic acid to yield a compound of formula (II) or a salt thereof, wherein R₁ has the same meaning as defined above; 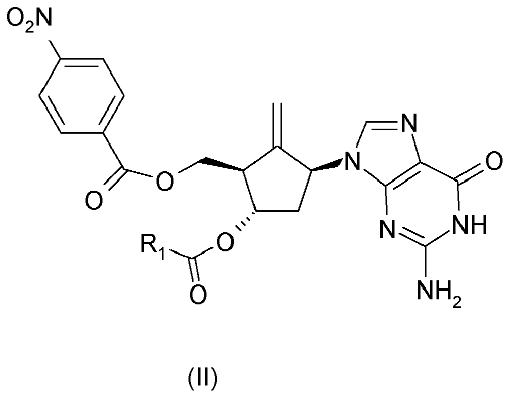
(b) submitting a compound of formula (II) to a deprotection reaction; and
(c) isolating the entecavir either as free base or as a pharmaceutically acceptable salt.

2. The process according to claim 1, wherein compounds of formula (II) and (III) are those where R₁ is a (C₁-C₄)-alkyl.

3. The process according to claim 2, wherein R₁ is methyl.

4. The process according to any of the claims 1-3, wherein the hydrolysis reaction is carried out at a temperature of between 40 °C and 80 °C.

5. The process according to any of the claims 1-4, wherein the deprotection reaction is carried out with a base selected from the group consisting of a metal alkaline (C₁-C₄)-alkoxide, a metal alkaline hydroxide, a metal alkaline carbonate and (1,8-diazabicyclo[5.4.0]undec-7-ene); or with a mixture of KCN/EtOH or NH₃/MeOH.

6. The process according to claim 5, wherein the base is a sodium (C₁-C₄)-alkoxide.

7. The process according to claim 6, wherein the base is sodium methoxide and the solvent is methanol.

8. The process according to any of the claims 1-7, further comprising a previous step of reacting a compound of formula (IV) wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro with a compound of formula (V) wherein X is selected from Cl, Br, and I, to yield a compound of formula (III).

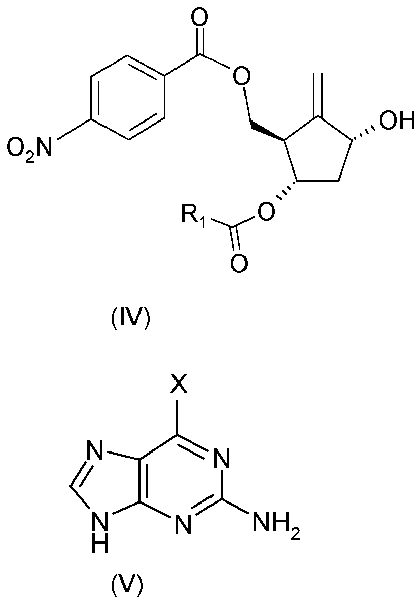

9. The process of claim 8, wherein in compound of formula (V) X is Cl.

10. The process according to any of the claims 7-9, further comprising a previous step of submitting a compound of formula (VI) wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro, to a desilylation reaction to remove the TBS group to yield a compound of formula (IV).

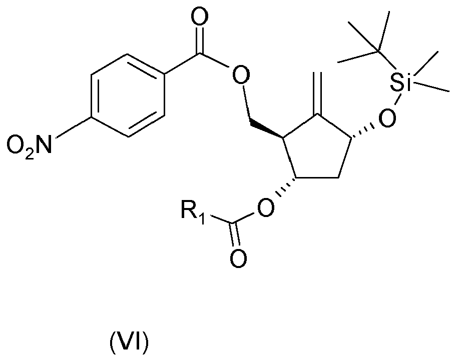

11. The process according to claim 10, further comprising a previous step wherein a compound of formula (VII) wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro is reacted with a compound of formula (VIII) wherein Y is an halogen or with p-nitrobenzoyl anhydride, to yield a compound of formula (VI).

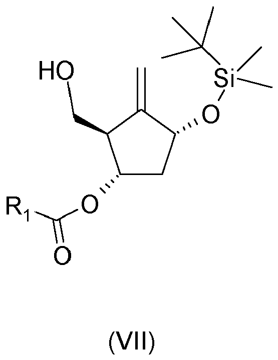

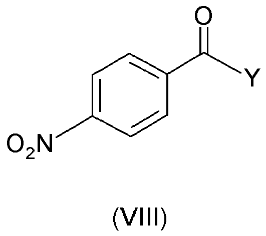

12. A compound of formula (II) or a salt thereof, wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

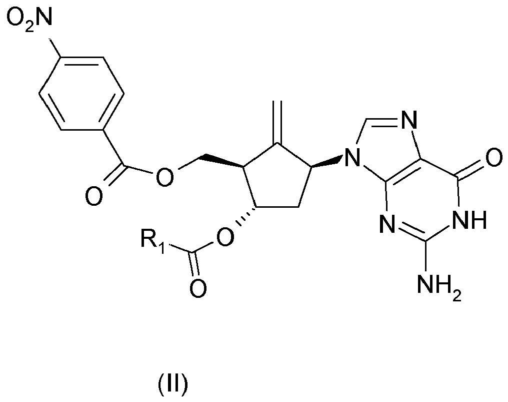

13. A compound of formula (III) or a salt thereof, wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro, and X is an halogen selected from Cl, Br, and I.

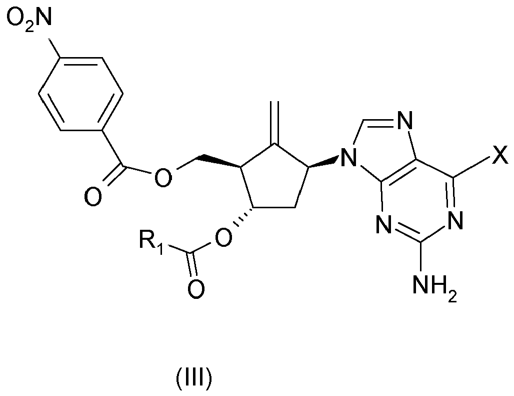

14. A compound of formula (IV), wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.

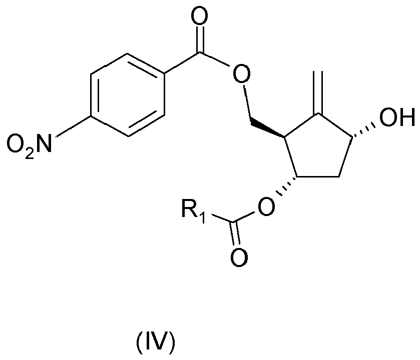

15. A compound of formula (VI), wherein R₁ is selected from the group consisting of (C₁-C₆)-alkyl, phenyl, and phenyl substituted by a radical selected from the group consisting of (C₁-C₄)-alkyl, (C₁-C₄)-alkoxy, halogen, and nitro.
